(19) Europäisches Patentamt
European Patent Office
Office européen des brevets

(11) Publication number: **0 257 992 B1**

(12) **EUROPEAN PATENT SPECIFICATION**

(45) Date of publication of patent specification: **12.08.92** (51) Int. Cl.[5]: **A61K 37/02**

(21) Application number: **87307383.7**

(22) Date of filing: **20.08.87**

(54) **Prophylactic and therapeutic agents for cerebral ischemia.**

(30) Priority: **20.08.86 JP 196006/86**

(43) Date of publication of application:
**02.03.88 Bulletin 88/09**

(45) Publication of the grant of the patent:
**12.08.92 Bulletin 92/33**

(84) Designated Contracting States:
**AT BE CH DE FR GB IT LI NL SE**

(56) References cited:

**MEDICAL BIOLOGY, vol. 62, 1984, pages 122-124; S. REHNCRONA: "Free radicals in brain ischemia"**

**ADVANCES IN NEUROLOGY, vol. 26, 1979, pages 285-286, Raven Press, New York, US; S. REHNCRONA et al.: "Cortical and cerebrospinal fluid concentrations of reduced and oxidized glutathione during and after cerebral ischemia"**

**PROC. NATL. ACAD. SCI., vol. 81, August 1984, pages 4732-4735; V.P. WELLNER et al.: "Radioprotection by glutathione ester: Transport of glutathione ester into human lymphoid cells and fibroblasts"**

(73) Proprietor: **YAMANOUCHI PHARMACEUTICAL CO. LTD.**
**No. 3-11 Nihonbashi-Honcho, 2-chome Chuo-ku**
**Tokyo(JP)**

(72) Inventor: **Yamamoto, Minoru**
**1370, Horiuchi Hayama-cho**
**Miura-gun Kanagawa(JP)**
Inventor: **Sakamoto, Nobuo**
**5-17-35, Matsuba**
**Ryugasaki-shi Ibaragi(JP)**
Inventor: **Nagano, Yoshinobu**
**15-14, Midori-cho 2-chome**
**Tanashi-shi Tokyo(JP)**

(74) Representative: **Geering, Keith Edwin et al**
**REDDIE & GROSE 16 Theobalds Road**
**London WC1X 8PL(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

ARCHIVES OF BIOCHEMISTRY AND BIOPHYISICS, vol. 239, no. 2, June 1985, pages 538-548, Academic Press Inc.; M.E. ANDERSON et al.: "Glutathione monoethyl ester: Preparation, uptake by tissues, and conversion to glutathione"

HEPATOLOGY, vol. 4, no. 4, 1984, pages 739-742, The American Association for the Study of Liver Diseases, US; A. MEISTER: "New developments in glutathione metabolism and their potential application in therapy"

## Description

The present invention relates to a prophylactic and therapeutic agent for cerebral ischemia comprising as an effective component glutathione monoalkyl ester of the following formula, or salt thereof:

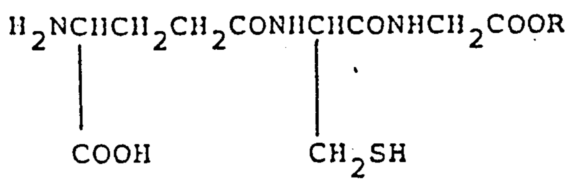

wherein R represents an alkyl group, preferably a lower alkyl group e.g. isopropyl.

Ischemia refers to a state in which blood is locally deficient. Ischemia in the brain is cerebral ischemia.

The whole cerebral blood flow in humans is estimated to be 50 to 60 ml/100g brain/min. on average. Thus, when the brain weight is 1400 g, the whole blood flow required is 700 to 840 ml/min. This indicates that the brain requires very large amounts of blood flow, considering that the weight ratio of the brain to the whole body is about 1 : 36 whereas the ratio of the cerebral blood flow to the heart rate is about 1 : 6.5.

Various factors affect the cerebral blood flow, such as perfusion pressure; carbon dioxide ($CO_2$) and oxygen ($O_2$) content and pH of the arterial blood or brain tissue; blood vicosity; cerebral pressure; cerebrovacular endothelial cell disorders. Whilst perfusion pressure to the brain can vary, the cerebrovascular tract has an automatic control ability to compensate for the variation and keep its blood flow constant.

However, disorders of this mechanism occur because of a variety of pathological conditions to cause the state of cerebral ischemia. Pathological conditions that cause such a cerebral ischemic state include cerebral thrombus, cerebral embolism, cerebral hemorrhage and subarachnoid hemorrhage.

As prophylactic and therapeutic agents for cerebral ischemic diseases, there have been hitherto applied anti-coagulants, thrombolytic agents and thrombocyte coagulation retardants for cerebral thrombus and embolism; thrombolytic agents for hemorrhagic conditions, steroid hormones for cerebral edema; and various agents for symptomatic therapy for clinical conditions.

In addtion cerebral metabolism activators such as cytochrome C, Meclofenoxate, Citicoline have been used for disorders of cerebral metabolism function.

However, the drugs conventionally used have insufficent direct effect on cerebral ischemia and none has been effective for alleviating hypoxia, especially in acute periods.

Accordingly, extensive investigations have been made to develop drugs exhibiting anti-cerebral ischemic action or anti-hypoxia and effective as prophylactic and therapeutic agents for cerebral ischemia. We have found, surprisingly, that the glutathione monoalkyl esters (I) possess marked effect for prevention and treatment of these cerebral ischemic diseases.

The glutathione monoalkyl esters (I) are alkyl esters of the glycine carboxyl group of glutathione. They have been reported, when de-esterified to free carboxyl form, to have detoxication activity and to protect cells from oxygen and its metabolites (for example peroxides), free radicals and externally given compounds. - see for example, Proc.Natl.Acad.Sci, vol.89, August 1984, pages 4732-4735. Their effectiveness for the prophylaxis and therapy of cerebral ischemic diseases was unknown heretofore. "Medical Biology", vol.62, 1984 at pages 122-124 discusses theories of free radical oxidative mechanisms for brain ischemia but is unable to conclude that this mechanism does operate; in particular, although glutathione is a free radical scavenger, brain ischemia was found not to induce any increase in its oxidised form or in the ratio of this oxidised form to glutathione.

Our EP Patent Application 87304962.1 is directed to anti-anemic agents comprising glutathione monoalkyl esters as effective component; this is clearly distinct from the present invention as regards both the diseases concerned and the mechanism by which the medicament acts.

In the glutathione monoalkyl esters (I) the alkyl group is a straight or branched group of e.g. 1 to 10 carbon atoms. Specific examples include methyl, ethyl, propyl, isopropyl, butyl, isobutyl, tert-butyl, pentyl, isopentyl, neopentyl, tert-pentyl, 1-methyl-butyl, hexyl, isohexyl, 2-methylpentyl, 1-ethylbutyl, heptyloctyl, nonyl and decyl groups.

The compound (I) and salts thereof can be produced, for example, by the following process:

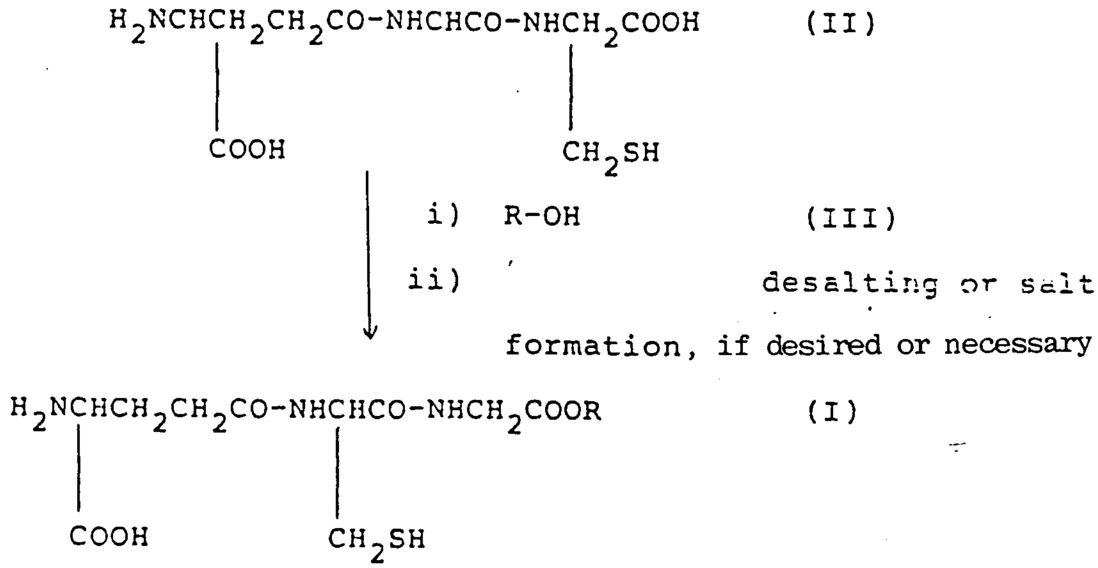


wherein R is as defined above.

Namely, the process can be carried out by reacting glutathione (II) with alcohol (III) in the presence of acid (for example, sulfuric acid) to produce glutathione monoalkyl ester salt with further desalting or salt formation treatment.

The compounds (I) are preferably administered in free form. They may also be administered in the form of salts with inorganic acids such as hydrochlorides, nitrates and sulfates or with organic acids such as oxalates, p-toluenesulfonates and maleates. When the compounds are administered in salt form they can be desalted or administered with addition of a base such as sodium hydrogencarbonate that does not adversely affect the living body.

The glutathione monoalkyl esters (I) and salts can be formulated as tablets, powders, granulates, granules, capsules, pills, liquids and injections, using conventional carriers, for oral or parenteral administration.

The dose may be varied depending upon administration route and body weight, age, condition etc. of the patient; an appropriate adult dose for intravenous injection is several tens to 600 mg daily.

The isopropyl ester (I) and salts have an acute toxicity ($LD_{50}$) of approximately 5.3 g/kg on intraperitoneal injection into mice.

Reference Example 1

Production of glutathione monoisopropyl ester:

(1) To 800 ml of isopropyl alcohol was added 124 g of glutathione. While stirring, 42 ml of 95% sulfuric acid was added dropwise to the mixture. Heat evolved but it was unnecessary to cool. About one hour later, the system became a homogeneous solution and glutathione monoisopropyl ester (isopropyl $\gamma$-L-glutamyl-L-cysteinyl glycinate) sulfate crystals began to precipitate about 24 hours after. The stirring was further continued overnight. The crystals were taken by filtration, washed with 200 ml of isopropyl alcohol and dried under reduced pressure to given 88.5 g of the sulfate. A part of the crystals was recrystallized from water-isopropyl alcohol mixture (mixing ratio 1:5) and purified to provide a sample for analysis.

Melting point: 145 - 150°C

Elemental analysis ( $C_{13}H_{23}N_3O_6S \cdot 1/2H_2SO_4 \cdot 1/2H_2O$)

| | C | H | N | S |
|---|---|---|---|---|
| Calcd. (%) | 38.32 | 6.18 | 10.31 | 11.80 |
| Found (%) | 38.11 | 6.34 | 10.32 | 12.10 |

(2) In 1.2 liters of water was dissolved 50.0 g of the unpurified sulfate obtained in (1). The solution was charged into 1.5 liters of HP-20, eluted with water and then eluted with a methanol-water mixture (mixing ratio 1:1) to given 2.5 liters of the fraction containing the product. After concentrating the fraction, the concentrate was freeze dried to give 33.8 g of glutathione monoisopropyl ester.

(i) Melting point: 184 - 186°C
(ii) Infrared absorption spectra (KBr, $cm^{-1}$)
1730, 1635, 1525, 1400, 1370, 1205, 1100
(iii) Specific rotary power ($[\alpha]_D^{21}$
-31.0 (c = 1.0, water)
(iv) Nuclear magnetic resonance spectra (CMSO-$d_6$, $\delta$ ppm)
1.20 (6H, d, J = 6Hz)
1.72 - 2.16 (2H, m)
2.20 - 2.40 (2H, m)
2.64 - 2.86 (2H, m)
3.20 - 3.56 (1H, m)
3.80 (2H, s)
4.20 - 4.60 (1H, m)
4.68 - 5.08 (1H, m)

Reference Example 2

Production of glutathione monoethyl ester:

Glutathione monoethyl ester (ethyl $\gamma$-L-glutamyl-L-cysteinyl glycinate) was produced in a manner similar to Reference Example 1.

(i) Infrared absoprtion spectra (KBr, $cm^{-1}$)
1735, 1635, 1520, 1400, 1200
(ii) Specific rotary power ($[\alpha]_D^{21}$
-27.2 (c = 1.0, water)
(iii) Nuclear magnetic resonance spectra (CMSO-$d_6$, $\delta$ ppm)
1.16 (3H, t, J = 7Hz)
1.70 - 2.04 (2H, m)
2.16 - 2.40 (2H, m)
2.60 - 2.82 (2H, m)
3.16 - 3.40 (1H, m)
3.80 (2H, s)
4.04 (2H, q, J = 7Hz)
4.20 - 4.48 (1H, m)

Example 2

(Injection Formulation)

Gluathione monoisopropyl ester sulfate was suspended in purified water in a concentration of 45 mg/ml and the suspension was cooled to 5°C or below. About 2-fold mols of sodium hydrogencarbonate was added to dissolve the ester sulfate. After adjusting to pH 4, the solution was diluted to a concentration of 40

mg/ml followed by aseptic filtration. The solution was charge in 25 ml lots into separate vials and freeze dried.

Example 2

Action of glutathione monoisopropyl ester on death of rats ligated at carotids (both sides).

Both carotids of male Wistar rats (weighting about 300 g) were permanently ligated to cause a cerebral ischemic state. The rats suffered neural conditions (paralysis or spasm of the limbs) and most of them died in 24 hours. The influence of the ester on death 24 hours afer ligation was examined. The ester was intraperitoneally administered immediately after the ligation, and 1 and 2 hours after the ligation. Physiological saline alone was given to the control group.

The results, evaluated by Fischer's assay method are shown in Table I.

Table I

| | Dose(1) | Number of Animals | Number of Dead Animals(2) |
|---|---|---|---|
| Control | | 24 | 21 |
| Glutathione monoisopropyl ester | 300 | 24 | 15* |

(1) mg/kg i.p.: amount of a single dose given.

(2) Number of dead animals 24 hours after ligation

*5% is a significant difference in death rate compared to the control group (group given physiological saline)

As shown in Table I, the number of dead animals in the gluathione monoisopropyl ester group was significantly smaller than in the control group. It is thus evident that the ester exhibits protective action against cerebral ischemia in rats having both carotids ligated.

Example 3

Action of gluathione monoisopropyl ester on survival time of low oxygen-loaded mice:

Male ICR mice (weighing about 30 g) were put in a glass vessel having a volume of 2 liters, equipped with an inhalation opening and an exhaust opening. A gaseous mixture of 95.3% nitrogen and 4.7% oxygen was blown into the vessel at 5 ℓ/min. to cause a low oxygen state, and the time for mice to die was measured. The ester was intravenously administered 10 minutes before the inhalation of the gaseous mixture. Only physiological saline was given to the control group.

Table II. Action on Survival Time of Low Oxygen-Loaded Mice

| | Dose (1) | Number of Animals | Survival Time (2) |
|---|---|---|---|
| Control | | 14 | 201±18 |
| Glutathione monoisopropyl ester | 300 | 14 | 262±22* |

(1) mg/kg i.p.: amount of a single dose given.

(2) Unit: second

* 5% is a significant difference in death rate as compared to the control group (group given physiological saline).

As shown in Table II, the survival time in the glutathione monoisopropyl ester group was significantly longer than in the control group. It is thus evident that the ester exhibits protective action against the low oxygen load.

Example 4

Action on neural conditions of autoblood-injected rats and malonedealdehyde content in the brain:

Male Wistar rats (weighing about 300 g) were anesthesized with pentobarbital (40 mg/kg i.e.) and fixed on a brain normal position fixing device. In order to form hematoma in the brain, 0.2 cc of autoblood previously collected from the tail vein was injected in the site of the brain at 4 mm to the right from the arrow suture, at 2 mm to the tail side from the crown suture and at 4 mm of the brain surface. The left rear paw was forced onto a stand having a height of 4.5 cm and the time from which the forced posture was maintained was measured and evaluated as neural condition. The ester was intraperitoneally administered immediately after the injection of autoblood and then once a day. Physiological saline alone was given to the control group. The measurement of neural condition was made after the operation daily for 4 consecutive days, 30 minutes after administration.

Immediately after the observation of the neural condition on the fourth day of the operation, the head of each rat was cut off and the right hemispherical brain was isolated. The lipid peroxides in the brain were fluorometrically determined by forming about 10% of a homogenate with 1.15% potassium chloride, taking 0.1 ml of the homogenate and measuring using malonedealdehyde as a standard substance according to Yagi's method.

The results, evaluated by the Student's t-test, are shown in Table III.

Table III. Action on Neural Condition of Rats Injected with Autoblood

| | | Duration (2) | | | |
|---|---|---|---|---|---|
| | Dose(1) | Day 1 | Day 2 | Day 3 | Day 4(3) |
| Control | | 42±10 | 53±12 | 43±11 | 31±10 |
| Glutathione monoisopropyl ester | 300 | 11±5* | 22±11 | 8±1** | 7±1* |

(1) mg/kg i.p.: amount of a single dose given.

(2) Unit: second

(3) Number of days after the operation.

*5% is a significant difference in duration as compared to the control group (group given physiological saline).

**1% is a significant difference in duration as compared to the control group. Number of animals: 10 - 11.

As shown in Table III, the period of maintaining the forced posture was significantly shorter in the glutathione monoisopropyl ester group as compared to the control group. It is thus evident that the ester exhibits action to improve the neural conditon of the autoblood-injected rats.

Futher in Table IV are shown the results of the effect on malonedealdehyde in the brain. The results were evaluated by the Student's t-test.

Table IV. Action on Malonedealdehyde in the Brain of Rats Injected with Autoblood

| | Dose (1) | Content of Malonedealdehyde (2) |
|---|---|---|
| Control | | 168±15 |
| Glutathione monoisopropyl ester | 300 | 97±20** |

(1) mg/kg i.p.

(2) Unit: nmol/g wet weight

** 1% is a significant difference as compared to the control group (group given physiological saline). The malonedealdehyde in the brain was 77 ± 3 nmol/g wet weight. Number of animals: 10 - 11.

As shown in Table IV, an increase of the malonedealdehyde content in the brain was noted in the autoblood-injected rats. The malonedealdehyde content in the brain was significantly lower in the glutathione monoisopropyl ester group as compared to the control group. It is thus evident that the ester inhibits the production of increased lipid peroxides in the autoblood-injected rats.

From the foregoing experimental results, it is noted that glutathione monoisopropyl ester exhibits the action of improving the neural conditions in the autoblood-injected rats, and the inhibition of production of lipid peroxides would be a part of the action.

## Claims

1. The use of glutathione monoalkyl ester of the following formula, or salt thereof:

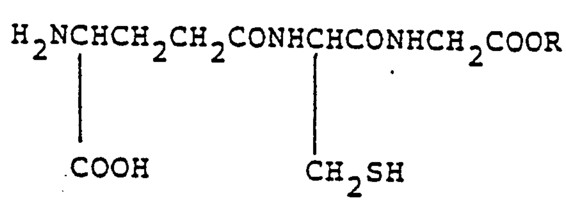

wherein R represents an alkyl group, for the preparation of a medicament for the prevention or treatment of cerebral ischemia or hypoxia.

2. The use as claimed in claim 1 wherein R is a $C_1$ to $C_{10}$ alkyl group.

3. The use as claimed in claim 1 wherein R is isopropyl.

## Revendications

**1.** L'utilisation d'ester monoalcoyle du glutathion de la formule suivante, ou un sel de celui-ci :

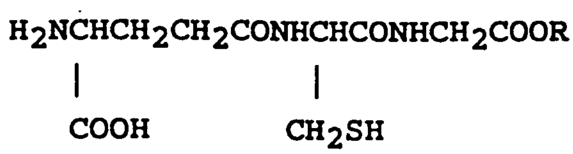

où R représente un groupement d'alcoyle, pour la préparation d'un médicament pour la prévention ou le traitement d'ischémie cérébrale ou d'hypoxie.

**2.** L'utilisation telle que revendiquée dans la revendication 1 où R est un groupement alcoyle en $C_1$ à $C_{10}$.

**3.** L'utilisation telle que revendiquée à la revendication 1 où R est l'isopropyle.

## Patentansprüche

**1.** Verwendung von Glutathion-monoalkylester der folgenden Formel, oder eines Salzes davon:

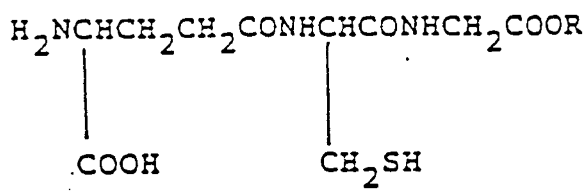

in der R eine Alkylgruppe bedeutet, zur Herstellung eines Arzneimittels zur Verhütung oder Behandlung von cerebraler Ischämie oder Hypoxie.

**2.** Verwendung nach Anspruch 1, wobei R eine $C_1$- bis $C_{10}$-Alkylgruppe ist.

**3.** Verwendung nach Anspruch 1, wobei R Isopropyl ist.